# EUROPÄISCHE PATENTSCHRIFT

(11) **EP 1 830 636 B1**
(45) Veröffentlichungstag und Bekanntmachung des Hinweises auf die Patenterteilung: **24.01.2018**
(21) Anmeldenummer: 05825981.3
(22) Anmeldetag: 20.12.2005
(51) Int. Cl.: A01N 31/02, A01N 25/16

(54) **ALKOHOLISCHER PUMPSCHAUM**
PUMPED FOAM CONTAINING ALCOHOL
MOUSSE A POMPER CONTENANT DE L'ALCOOL

(30) Priorität: 21.12.2004 DE 102004062775
(43) Veröffentlichungstag der Anmeldung: 12.09.2007
(73) Patentinhaber: Deb IP Limited, Denby, Derbyshire DE5 8JZ (GB)
(72) Erfinder: VEEGER, Marcel, 47574 Goch (DE); HIMMING, Markus, 46045 Oberhausen (DE)
(74) Vertreter: Elsy, David
(86) Internationale Anmeldenummer: PCT/EP2005/013742
(87) Internationale Veröffentlichungsnummer: WO 2006/066888

(56) Entgegenhaltungen:
- WO-A-03/028671
- US-A- 4 511 486
- US-A- 5 167 950
- US-A1- 2002 127 253
- SANDERS P A: "AQUEOUS ALCOHOL AEROSOL FOAMS" DRUG AND COSMETIC INDUSTRY, Bd. 99, Nr. 2, 1996, Seiten 56,58,60,142,143,146-154, XP000960450

## Beschreibung

Die Erfindung betrifft alkoholische Schaumzusammensetzungen, insbesondere Pumpschäume, und deren Verwendung als Desinfektionsmittel.

Desinfektionsmittel dienen der Bekämpfung pathogener Mikroorganismen wie z.B. Bakterien, Viren, Sporen, Pilze etc. und ihr Einsatz ist in vielen Bereichen unverzichtbar bzw. deren Verwendung wird in vielen Ländern vom Gesetzgeber ausdrücklich verlangt.

Üblicherweise werden Desinfektionsmittel entsprechend ihres Einsatzgebietes gegliedert und man unterscheidet je nach Verwendungszweck Antiseptika zur Wund-, Haut-, Stuhl- und Sputumdesinfektion sowie Instrumentendesinfektion, Wäsche- und Flächendesinfektionsmittel, insbesondere aber auch Haut- und Händedesinfektionsmittel.

Das Anwendungsgebiet der vorgenannten Desinfektionsmittel ist medizinisch indiziert und dient der Prävention von Infektionen in Krankenhäusern, Arzt- und Zahnarztpraxen, in öffentlichen Bereichen wie Schulen, Kindergärten und Pflegeeinrichtungen z.B. Altenheimen, Sanatorien etc., aber auch Sportstätten sowie in anderen Bereichen, in denen Infektionen übertragen werden können. Neben dem Einsatz von Desinfektionsmitteln in der Lebensmittelindustrie und Pharmaindustrie ist nicht nur allgemein deren Einsatz am Arbeitsplatz oder in Wohnungen, sondern auch deren Verwendung in Dienstleistungseinrichtungen wie z.B. Wäschereien und Küchen zu nennen, deren Produkte direkt an Patienten oder Verbraucher geliefert wird.

Der Nachweis der Wirksamkeit solcher desinfizierender Mittel für eines oder mehrere der oben genannten Anwendungsgebiete erfolgt nach eingehender Prüfung dieser Mittel unter Zugrundelegung normierter Prüfverfahren, die z.B. in Deutschland den Richtlinien der Deutschen Gesellschaft für Hygiene und Mikrobiologie (DGHM) bzw. den Richtlinien des französischen Normungsinstitut AFNOR (Association Française de Normalisation) entnommen sind. Beispielhaft sei auf die nachfolgenden Normen hingewiesen:
- DIN EN 1040: Chemische Desinfektionsmittel und Antiseptika (Basistest)
- DIN EN 1276: Chemische Desinfektionsmittel und Antiseptika Bakterizide Wirkung in den Bereichen Lebensmittel, Industrie, Haushalt und öffentliche Einrichtungen
- DIN EN 1499: Desinfizierende Händewaschung
- DIN EN 1500: Hygienische Händedesinfektion
- DIN EN 12054: Chemische Desinfektionsmittel und Antiseptika Produkte für die hygienische und chirurgische Händedesinfektion und Händewaschung - Bakterizide Wirkung
- DIN EN 12791: Chirurgische Händedesinfektionsmittel
- AFNOR T 72 300: Bakterizide Wirksamkeit von Antiseptika und Desinfektionsmitteln, die als Flüssigkeit, gemischt in Wasser angewendet werden
- AFNOR T 72 170: Bakterizide Wirksamkeit in Gegenwart von Störstoffen
- NF EN 1040: Bakterizide Wirksamkeit von Antiseptika und chemischen Desinfektionsmitteln
- NF EN 1275: Fungizide Wirksamkeit von Antiseptika und chemischen Desinfektionsmitteln

Antiseptika unterliegen als Arzneimittel darüber hinaus gesetzlich geregelter Zulassungs- und Registrierungsverfahren.

Wie z.B. der DE-A-43 28 828 entnommen werden kann, gibt es verschiedene Verfahren zur Durchführung der Händedesinfektion. Explizit werden dort die in Deutschland übliche alkoholische Händedesinfektion sowie die waschende Händedesinfektion (Scrub-Methode) genannt. Produkte, die u.a. für die Händedesinfektion bestimmt sind, müssen wenigstens den in den oben genannten Normen aufgeführten Mindestanforderungen genügen, wenn sie entsprechend der erfüllten Normen zertifiziert und als Präparate in der Desinfektionsliste der DGHM aufgenommen werden sollen.

Im Markt erhältliche Desinfektionsmittel, insbesondere Haut- und Händedesinfektionsmittel bestehen üblicherweise aus Alkohol oder Mischungen aus Alkoholen sowie optional Pflegewirkstoffen, die nach dem Verdunsten der Alkoholkomponente auf der Haut verbleiben, z.B. nichtflüchtige antimikrobiell wirksame Substanzen und/oder übliche Hautpflegestoffe sowie gegebenenfalls weiteren Hilfsstoffen. Dient lediglich die Alkoholkomponente als antimikrobieller Wirkstoff, so ist der Alkoholgehalt in dem Mittel so zu wählen, daß auch bei Verdunsten eines Teiles des Alkohols eine desinfizierende Wirkung gewährleistet ist. In diesem Zusammenhang ist bekannt, daß dies für ethanolische Zusammensetzungen mit einem Alkoholgehalt von mindestens 52 Gew. -% der Fall ist.

Im Hinblick auf die Nachteile alkoholischer Desinfektionslösungen bei der Applikation für die Haut- und Händedesinfektion, insbesondere aufgrund der schwierigen Dosierung, weil die benötigte Desinfektionsmittelmenge vielfach nicht gleichmäßig über die Haut bzw. die Hände verteilt werden kann und auch wässrige Alkohollösungen sehr leicht von den Händen tropfen, sind solchen Desinfektionslösungen Verdickungsmittel zugesetzt worden, um die Viskosität dieser Mittel zu erhöhen. Als Stand der Technik ist hier beispielhaft die EP-B-0 604 848 zu nennen, die eine schnelltrocknende Desinfektionsmittelzusammensetzung zum Gegenstand hat. Als Verdickungsmittel wird eine Kombination aus Carboxyvinylpolymeren und Hydroxypropylmethylcellulose beschrieben, wobei das Gesamtgewicht der beiden Komponenten in der Desinfektionsmittelzusammensetzung nicht größer als 3 Gew.-% ist.

Weiterhin sind im Stand der Technik antimikrobielle alkoholische Gelzusammensetzungen zur Haut- und Händedesinfektion bekannt, die Feuchthaltemittel und hautpflegende Substanzen enthalten, wie sie z.B. in der US 4 956 170 beschrieben worden ist. In diesen Zusammensetzungen werden vernetzte teilneutralisierte bzw. neutralisierte Acrylsäurepolymeren als Verdickungsmittel eingesetzt. Als antibakterielles Mittel dient in diesen Zusammensetzungen 60 bis 75 Gew.-% Alkohol wie z.B. Ethanol, Isopropropylalkohol oder Mischungen hiervon. Im Hinblick auf die in diesen Gelzusammensetzungen enthaltenen Weichmacher (Emollientien), insbesondere Petrolatum und andere Mineralölprodukte, die in kosmetischen Präparaten eingesetzt werden können sowie weiteren hydrophoben Inhaltsstoffen, die in Kosmetika aber auch in Desinfektionsmittel unbedenklich eingesetzt werden können, ist gefunden worden, daß die Verwendung solcher Inhaltsstoffe in alkoholischen Gelzusammensetzungen mit hohem Alkoholgehalt für die Stabilität dieser Gele sehr nachteilig ist, weil die Gele bei der Lagerung im Laufe der Zeit ihre Viskosität und damit ihre Stabilität verlieren und die Zusammensetzungen zerfließen. Allgemein wurde festgestellt, daß die Gelstabilität mit zunehmendem Alkoholgehalt leidet, insbesondere bei Alkoholgehalten über 60 Gew.-%.

Solch hohe Alkoholgehalte, insbesondere in Gelzusammensetzungen mit Alkohol als einziger Wirkkomponente sind jedoch unverzichtbar, damit solche Mittel auch als Desinfektionsmittel für die Händedesinfektion zertifiziert werden können.

In der DE 101 32 382 ist ein einfaches, wirtschaftliches Herstellungsverfahren zur Herstellung von stabilen desinfizierenden Haut- und Handpflegegelen mit hohem Alkoholgehalt beschrieben worden, das die Herstellung von desinfizierenden Handpflegegelen mit pflegenden Komponenten ermöglicht, die ohne weitere zusätzliche antimikrobielle Zusatzstoffe u.a. die Normen
- DIN EN 1499: Desinfizierende Händewaschung
- DIN EN 1500: Hygienische Händedesinfektion
erfüllen und darüberhinaus eine Hepatitis B Wirkung aufweisen. Obgleich sich gezeigt hat, daß die Applikation eines desinfizierenden Mittels in Gelform der Applikation eines desinfizierenden Mittels in flüssiger Form, insbesondere im Hinblick auf dessen Tropfungsneigung vorzuziehen ist, haben nichtsdestoweniger solche desinfizierenden alkoholischen Gelzusammensetzungen den Nachteil, daß sie beim Auftragen auf die Haut ihre Gelstruktur verlieren müssen, um eine gleichmäßige Benetzung der Hautpartien und damit eine sichere Desinfektionswirkung zu gewährleisten.

Weiterhin sind im Stand der Technik alkoholische Reinigungsschaumzusammensetzungen bekannt, die über im Markt erhältliche Pumpschaumsysteme abgegeben werden, die vornehmlich in sanitären Anlagen von Krankenhäusern, Arzt- und Zahnarztpraxen, Schulen, Kindergärten und Pflegeeinrichtungen, wie z.B. Altenheimen, Sanatorien etc. anzutreffen sind. Der Alkoholgehalt solcher Schaumzusammensetzungen liegt jedoch lediglich bei 40 Gew.-%, weil bei höheren Alkoholgehalten die Instabilität der Schäume wächst. Dies kann als Grund dafür angesehen werden, daß die vorteilhafte Applikationsform mittels Schaum, die in einer noch besseren Handhabbarkeit im Vergleich zu alkoholischen Gelen liegt, bisher noch nicht für Desinfektionsmittel, insbesondere noch nicht für die Haut- und Händedesinfektion aufgrund des geringen Alkoholgehaltes der bisher im Markt erhältlichen Produkte in Betracht kam.

US5167950 beschreibt antimikrobielle Schaumformulierungen, enthaltend Ethanol oder Isopropanol sowie polymere Verdickungsmittel.

P.A. Sanders (D&CI/August 1966, 56-62, 142-154, XP000960450) beschreibt Formulierungsbedingungen für alkoholische Aerosolschäume. Aufgabe der vorliegenden Erfindung war es daher, alkoholische Schaumzusammensetzungen bereitzustellen, die insbesondere als Pumpschäume über übliche Pumpschaumsysteme an den Verbraucher zu Desinfektionszwecken, vorzugsweise zur Haut- und Händedesinfektion abgegeben werden können. Hierbei sollen die über das Pumpschaumsystem abgegebenen alkoholischen Schäume dergestalt stabilisiert sein, daß alkoholische Schäume mit einem Alkoholgehalt von mindestens 52 Gew.-% , insbesondere über 65 Gew.-% Alkohol abgegeben werden können, um eine sichere Desinfektion, insbesondere Haut- und Händedesinfektion zu gewährleisten.

Die Aufgabe wird gelöst durch eine alkoholische Schaumzusammensetzung zur Desinfektion, insbesondere Pumpschaumformulierung, umfassend die Komponenten
a.) mindestens 52 bis ≤ 99 Gew.-%, bezogen auf die Gesamtmenge der schäumbaren Zusammensetzung, eines Alkohols, der aus Methanol, Ethanol, 1-Propanol, 2-Propanol, 1-Butanol, Isobutylalkohol, tert.-Butylalkohol, die Amylalkohole 1-, 2-, 3- Pentanol, Neopentylalkohol, 1-Hexanol und Mischungen davon ausgewählt ist,
b.) 0,5 bis 20 Gew.-%, bezogen auf die Gesamtmenge der schäumbaren Zusammensetzung, mindestens eines Silikontensids, wobei das Silikontensid aus Polysiloxan-Polyether-Copolymeren ausgewählt ist;
c.) 0,01 bis 3 Gew.-%, bezogen auf die Gesamtmenge der schäumbaren Zusammensetzung, mindestens eines Polyalkylenglykols;
d.) mindestens ein Schaumstabilisierungsmittel das aus der Gruppe bestehend aus Celluloseether ausgewählt ist;
e.) mindestens ein Element, das aus der Gruppe bestehend aus kosmetischen Hilfs-, Zusatz- und/oder Wirkstoffen sowie Wasser oder einer Mischung davon ausgewählt ist, wobei die Oberflächenspannung der Komponente b) im Bereich ± 15 dyn/cm der Oberflächenspannung der Komponente a.) liegt oder der Oberflächenspannung der Komponente a.) entspricht und die Summe der Komponenten a.) bis e.) 100 Gew.-%, bezogen auf die Gesamtmenge der Schaumzusammensetzung, ist.

Es war völlig überraschend, daß solche alkoholischen Schaumzusammensetzungen, die vorzugsweise für die Haut- und Händedesinfektion geeignet sind, mit mindestens 52 Gew.-%, bezogen auf die Gesamtmenge der Schaumzusammensetzung, als Schaum über übliche Pumpschaumsyteme abgegeben werden können, ohne daß die Schäume aufgrund ihres hohen Alkoholgehaltes in der Zusammensetzung in sich zusammenfallen. Insbesondere wäre bei solch hohen Alkoholgehalten zu erwarten gewesen, daß die Alkoholkomponente solcher Schaumzusammensetzungen bei einem Alkoholgehalt höher 50 Gew.-% lediglich als Lösungsmittel wirkt, wodurch die grenzflächenaktiven Wirkungen der Tenside und damit verbunden deren Schäumungsvermögen herabgesetzt werden. Solches wurde jedoch nicht beobachtet. Vielmehr zeigte es sich, daß mit den erfindungsgemäßen Schaumzusammensetzungen in üblichen Pumpschaumsystemen stabile voluminöse Schäume für Desinfektionszwecke herstellbar waren. Erfindungsgemäß bevorzugt liegt die Oberflächenspannung der Komponente b) im Bereich von ≥ 20 bis ≤ 40 dyn/cm. Die erfindungsgemäße alkoholische Schaumzusammensetzung enthält als Komponente a.) einen Alkohol, der aus. Methanol, Ethanol, 1-Propanol, 2-Propanol, 1-Butanol, Isobutylalkohol, tert. Butylalkohol, die Amylalkohole 1-, 2-, 3- Pentanol oder Neopentylalkohol sowie 1-Hexanol und Mischungen davon, ausgewählt ist, wobei Ethanol als Komponente a.) besonders bevorzugt ist. Die erfindungsgemäße Schaumzusammensetzung enthält 52 bis 99 Gew.-%, vorzugsweise 55 bis 96 Gew.-% und insbesondere mehr als 60 bzw. 65 Gew.-% Ethanol. Besonders vorteilhaft ist hinsichtlich der Desinfektionswirkung, daß die erfindungsgemäßen alkoholischen Schäume mehr als 80 Gew.-% bzw. 90 Gew.-% Alkohol enthalten können.

Als Komponente b.) enthalten die erfindungsgemäßen Schaumzusammensetzungen mindestens ein Silikontensid, wobei das Silikontensid aus Polysiloxan-Polyether-Copolymeren ausgewählt ist, mit der Maßgabe, daß die Oberflächenspannung des in der Schaumzusammensetzung enthaltenden Tensides oder des Tensidgemisches im Bereich ± 15 dyn/cm der Oberflächenspannung der Komponente a.), d.h. der Alkoholkomponente liegt oder der Oberflächenspannung der Komponente a.) entspricht.

Die Gesamtmenge des Tensides oder Tensidgemisches beträgt 0,5 bis 20, vorzugsweise 1 bis 10 und besonders bevorzugt 2 bis 5 Gew.-%, bezogen auf die Gesamtmenge der Schaumzusammensetzung.

Solche Tenside sind Polysiloxan-Polyether-Copolymere [INCI(CFTA): Dimethicone Copolyol], die von der Firma Goldschmidt AG, Essen, unter der Handelsbezeichnung ABIL® erhältlich sind, insbesondere Polysiloxan-Polyether-Copolymere der Produktreihe B 88, wie z.B. ABIL® B 8843, ABIL® B 8851, ABIL® B 8852, ABIL® B 8863, ABIL® B 88183 und ABIL® B 88184. Besonders bevorzugt enthalten die erfindungsgemäßen Schaumzusammensetzungen Polysiloxan-Polyether-Copolymere als Komponente b.), die unter der Handelsbezeichnung ABIL® B 8832 (Bis-PEG/PPG-20/20 Dimethicone) erhältlich sind.

Weiterhin enthalten die erfindungsgemäßen Schaumzusammensetzungen als Komponente c.) mindestens ein Polyalkylenglykol, in Mengen von 0,01 bis 3, insbesondere 0,01 bis 0,2 und besonders bevorzugt 0,05 bis 0,2 Gew.-%, bezogen auf die Gesamtmenge der Schaumzusammensetzung. Erfindungsgemäß bevorzugte Polyalkylenglykole sind insbesondere Polyethylenoxid-Homopolymere mit einem Molekulargewicht von 100 000 bis 8 000 000, die als Handelsprodukt unter der Marke Polyox ® kommerziell erhältlich sind, wie beispielsweise Polyox ® WSR N-10, Polyox ® WSR N-80 (PEG-5M), Polyox ® WSR N-750 (PEG-7M), Polyox ® WSR N-3000 (PEG-14M), Polyox ® WSR N-3333, Polyox ® WSR-205 (PEG-14M), Polyox ® WSR-1105, Polyox ® WSR N-12K, Polyox ® WSR N-60K (PEG-45M)sowie Polyox ® WSR-301.

Die erfindungsgemäßen Schaumzusammensetzungen enthalten mindestens ein Schaumstabilisierungsmittel ausgewählt aus der Gruppe bestehend aus Celluloseether. Sie können zu 0,01 bis 20 Gew.-%, vorzugsweise 0,01 bis 10 Gew.-%, besonders bevorzugt 0,05 bis 3 Gew.-%, bezogen auf die Gesamtmenge der Schaumzusammensetzung, als Komponente d.) im Schaum enthalten sein. Die Schaumstabilisierungsmittel sind Celluloseether, wie z.B. Carboxymethylcellulose, Ethylcellulose, Hydroxypropylcellulose, Methylcellulose, Cellulosemischether beispielsweise Carboxymethylhydroxyethylcellulose, Ethylhydroxyethylcellulose, Methoxyhydroxyalkylcellulosen, Methylhydroxyalkylcellulosen, wie z.B. Methylhydroxyethylcellulose, Methylhydroxypropylcellulose, Methylhydroxybutylcellulose. Erfindungsgemäß bevorzugt sind Alkylcellulosen, insbesondere Methylcellulose und Ethylcellulose, die kommerziell unter den Handelsbezeichnungen Methocel® bzw. Ethocel® erhältlich sind.

Neben Wasser können die erfindungsgemäßen alkoholischen Schäume Hilfs-, Zusatz- und/oder Wirkstoffe wie z.B. Farbstoffe, Lösungsvermittler, Komplexbildner, Sequestrierungsmittel, Lichtschutzfilter oder Parfüm- bzw. Duftstoffe, pH-Regulatoren, Stabilisatoren, vorzugsweise Cetearylalkohol und/oder hydrierte Ricinusöle, wie z.B. Trihydroxystearin, Konservierungsmittel, Antioxidantien und/oder ölige oder wässrige Pflegekomponenten als Komponente e.) enthalten, insbesondere in üblichen Mengen von vorzugsweise 0,05 bis 5 Gew.-% bezogen auf das Gesamtgewicht der Schäume. Diese Bestandteile der Komponente e.) können üblicherweise in Mengen von 0 bis 5 Gew.-% bezogen auf das Gesamtgewicht des Schaumes, enthalten sein, wobei der Fachmann den Gewichtsanteil der Komponente e.) so wählt, daß es zu keiner Beeinträchtigung der Schaumbildung kommt.

In einer bevorzugten Ausführungsform der Erfindung enthalten die erfindungsgemäßen Schäume 55 bis 96 Gew.-% Ethanol, 1 bis 10 Gew.-% Bis-PEG/PPG-20/20 Dimethicone als Silikontensid, 0,05 bis 3 Gew.-% Ethylcellulose-Polymer als Stabilisierungsmittel in Kombination mit einem PEG-Polymer, ausgewählt aus PEG 7M - PEG 45M, vorzugsweise 0,05 bis 2 Gew.-%, wodurch in üblichen Pumpschaumsystemen sehr gute desinfizierende Schäume mit hervorragender Stabilität erhalten werden.

Die erfindungsgemäßen alkoholischen Schäume können besonders vorteilhaft als Desinfektionsmittel Verwendung finden, beispielsweise als Antiseptika zur Wund-, Haut-, Stuhl- und Sputumdesinfektion sowie Instrumentendesinfektion, als Wäsche- und Flächendesinfektionsmittel sowie erfindungsgemäß besonders bevorzugt als Haut- und Händedesinfektionsmittel.

Der mögliche optionale Zusatz von nichtflüchtigen antimikrobiell wirksamen Substanzen in den erfindungsgemäßen Schäumen dient insbesondere dazu, die desinfizierenden Eigenschaften der Alkoholkomponente zu verstärken. Dies ist jedoch davon abhängig, für welches Anwendungsgebiet die erfindungsgemäßen Schäume bestimmt sind.

Als gegebenenfalls einzusetzende antimikrobiell wirksame Substanzen, die vorzugsweise allein oder als Kombination mehrer desinfizierender Wirkstoffe in den erfindungsgemäßen Schäumen enthalten sein können, sind insbesondere Invertseifen, wie z.B. Kationtenside, Quartäre Ammoniumverbindungen u.a. Benzalkoniumchloride oder Benzethoniumchlorid, Biguanidverbindungen wie z.B. Chlorhexidin-Salze, Phenolverbindungen, Kresole, Perverbindungen, lodverbindungen, z.B. Polyvidon-lod, organische Säuren etc. zu nennen.

Nichtsdestoweniger ist der Zusatz solcher antimikrobiellen Wirkstoffe nicht notwendig, da die erfindungsgemäßen Schäume einen so hohen Alkoholgehalt aufweisen, so daß die Alkoholkomponente allein als desinfizierender Wirkstoff dient.

Pflege- und/oder Feuchthaltewirkstoffe, die optional in den erfindungsgemäßen Schäumen, insbesondere für die Verwendung der erfindungsgemäßen Schäume als Haut- und Händedesinfektionsmittel enthalten sein können, sind erfindungsgemäß Wirkstoffe, die nach dem Verdunsten der Alkoholkomponente des Schaumes auf der Haut verbleiben, beispielsweise übliche Hautpflegestoffe wie z.B. Dexpanthenol, Glyzerin, 1,2-Propandiol, Sorbitol, 1,3- Butylenglykol, Polyethylenglykol und andere Polyalkohole, Hyaluronsäuren, Harnstoff, Kamillenextrakte, alkoxylierte Cetylalkohole und/oder nichtflüchtige antimikrobiell wirksame Substanzen.

Da der hohe Alkoholanteil in den erfindungsgemäßen Schäumen bei Verwendung als Haut- und Händedesinfektionsmittel beim Auftragen eine Austrocknung der damit behandelten Hautpartien bewirkt, ist der Einsatz von mindestens einem Hautpflegestoff und/oder ein Feuchthaltewirkstoff im Hinblick auf die häufige Applikation solcher Desinfektionsmittel in der täglichen Praxis eigentlich unabdingbar.

Vorteilhaft für die Verwendung der erfindungsgemäßen Schäume zur Haut- und Händedesinfektion ist weiterhin ein Bestandteil von natürlichen pflanzlichen Gerbstoffen wie z.B. Frauenmantelkraut (Alchemilla xanthochlora, Rosaceae), Tormentillwurzelstock (Potentilla erecta, Rosaceae), Eichenrinde (Quercus petraea und Quercus robur, Fagaceae) Ratanhiawurzel (Krameria lappacea syn. K. triandra, Krameriaceae), Hamamelisblätter (Hamamelis virginiana, Hamamelidaceae) und Blaubeeren (Vaccinium myrtillus, Ericaceae) und natürlichen synthetischen Gerbstoffen, wie z.B. Na- bichlorophenylsulfamin, vorzugsweise in einer Menge von 0,01 bis 5 Gew.-% Aktivsubstanz, bezogen auf die Gesamtmenge der Schäume, wobei Hamamelis virginiana als Gerbstoff besonders bevorzugt ist.

Die erfindungsgemäßen Schäume zeichnen sich trotz ihres hohen Alkoholgehaltes durch eine sehr gute Stabilität aus, so daß durch die hier vorliegende Erfindung stabile desinfizierende Schäume, insbesondere zur Haut- und Händedesinfektion bereitgestellt sind, die ohne weitere zusätzliche antimikrobielle Zusatzstoffe u.a. die Normen
- DIN EN 1499: Desinfizierende Händewaschung
- DIN EN 1500: Hygienische Händedesinfektion
erfüllen und darüber hinaus eine Hepatitis B Wirkung aufweisen. Insbesondere die letztere Wirkung ist besonders vorteilhaft, da das Hepatitis B-Virus wie das HIV-Virus, das für die Verbreitung von AIDS (Acquired Immune Deficiency Syndrome) verantwortlich ist, übertragen wird, aber stabiler und infektiöser als das HIV-Virus ist. Daher sind alle Vorsichtsmaßnahmen gegen eine Übertragung von Hepatitis B auch präventiv gegen das HIV-Virus (vgl. Deutsches Ärzteblatt 84, Heft 18, S. B 874 vom 30.04.1987).

Da die erfindungsgemäßen Schäume Alkoholgehalte > 70 Vol.-%, bzw. 65 Gew.-%, mindestens aber 52 Gew.-% bezogen auf die Gesamtmenge des Schaumes, aufweisen, besteht somit auch eine Virusaktivität gegenüber "nackten", d.h. nicht "umhüllten" Viren, wie z.B. Polio- und Adenoviren, weshalb solche alkoholischen Schäume als Applikationsform, insbesondere für die Hautdesinfektion von besonderem Interesse sind.

Vorteilhaft ist weiterhin auch, daß sich die erfindungsgemäßen Schäume, insbesondere als Pumpschäume über übliche Pumpschaumsysteme an den Verbraucher zu Desinfektionszwecken, vorzugsweise zur Haut- und Händedesinfektion abgegeben werden können, insbesondere auch weil solche Pumpschäume üblicherweise preiswerter und einfacher in der Herstellung sind als Schäume auf Aerosolbasis. Exemplarisch können hier die im Markt erhältlichen Pumpschaumsysteme der Firmen Airspray (Niederlande), Keltec (Niederlande), Ophardt (Deutschland) und Supermatic (Schweiz) sowie Brightwell (Vereinigtes Königreich) genannt werden.

Erfindungsgemäß bevorzugte Schaumzusammensetzungen (alle Angaben in Gew.-%, bezogen auf die Gesamtmenge der Schaumzusammensetzung):

| Beispiel | A | B | C | D | E |
|---|---|---|---|---|---|
| Alkohol oder Alkoholmischung | 55,0 | 60,0 | 70,0 | 80,0 | 90,0 |
| Polyethyelenglykol-Homoploymer | 0,1 | 0,1 | 0,1 | 0,1 | 0,05 |
| Stabilisierungsmittel | 0,2 | 0,2 | 0,2 | 0,2 | 0,1 |
| Tensid oder Tensidmischung | 3,0 | 3,0 | 4,0 | 4,0 | 3,0 |
| Wasser | 41,7 | 36,7 | 25,7 | 15,7 | 6,85 |

Erfindungsgemäß bevorzugte Schaumzusammensetzungen zur Haut- und Händedesinfektion (alle Angaben in Gew.-%, bezogen auf die Gesamtmenge der Schaumzusammensetzung):

| Beispiel | 1 | 2 | 3 |
|---|---|---|---|
| Ethanol | 55,0 | 80,0 | 90,0 |
| PEG-14M | 0,1 | 0,1 | 0,05 |
| Ethylcellulose | 0,2 | 0,2 | 0,1 |
| Bis-PEG/PPG-20/ 20Dimethicone | 3,0 | 4,0 | 3,0 |
| Wasser demin. | 41,7 | 15,7 | 6,85 |

## Patentansprüche

1. Alkoholische Schaumzusammensetzung zur Desinfektion umfassend:
a) 52 bis ≤ 99 Gew.-%, bezogen auf die Gesamtmenge der schäumbaren Zusammensetzung, eines Alkohols, der aus Methanol, Ethanol, 1-Propanol, 2-Propanol, 1-Butanol, Isobutylalkohol, tert.-Butylalkohol, die Amylalkohole 1-, 2-, 3-Pentanol, Neopentylalkohol, 1-Hexanol und Mischungen davon ausgewählt ist;
b) 0,5 bis 20 Gew.-%, bezogen auf die Gesamtmenge der schäumbaren Zusammensetzung, mindestens eines Silikontensids, wobei das Silikontensid aus Polysiloxan-Polyether-Copolymeren ausgewählt ist;
c) 0,01 bis 3 Gew.-%, bezogen auf die Gesamtmenge der schäumbaren Zusammensetzung, mindestens eines Polyalkylenglykols;
d) mindestens ein Schaumstabilisierungsmittel das aus der Gruppe bestehend aus Celluloseether ausgewählt ist;
e) mindestens ein Element, das aus der Gruppe bestehend aus kosmetischen Zusatzstoffen, Hilfsstoffen, Wirkstoffen und Wasser oder einer Mischung davon ausgewählt ist, wobei die Oberflächenspannung der Komponente b) im Bereich ± 15 dyn/cm der Oberflächenspannung der Komponente a) liegt oder der Oberflächenspannung der Komponente a) entspricht und die Summe der Komponenten a) bis e) 100 Gew.-%, bezogen auf die Gesamtmenge der Schaumzusammensetzung, ist.

2. Alkoholische Schaumzusammensetzung nach einem der Ansprüche 1, wobei 52 bis ≤ 99 Gew.-%, bezogen auf die Gesamtmenge der Zusammensetzung, Ethanol allein oder als Teil der Komponente a) in dem Schaum enthalten sind.

3. Alkoholische Schaumzusammensetzung nach Anspruch 1 oder 2, **dadurch gekennzeichnet, daß** mindestens 80 bzw, 90 Gew. %, bezogen auf die Gesamtmenge der schäumbaren Zusammensetzung, Ethanol als Komponente a) in der schäumbaren Zusammensetzung enthalten sind.

4. Alkoholische Schaumzusammensetzung nach einem der Ansprüche 1 bis 3, **dadurch gekennzeichnet, daß** 0,01 bis 10 Gew.-%, bezogen auf die Gesamtmenge der schäumbare Zusammensetzung, mindestens ein Schaumstabilisierungsmittel als Komponente d) in der schäumbare Zusammensetzung enthalten sind.

5. Alkoholische Schaumzusammensetzung nach Anspruch 1, wobei:
die Komponente a) 55 bis 96 Gew.-%, bezogen auf die Gesamtmenge der schäumbaren Zusammensetzung, Ethanol ist;
die Komponente b) 1 bis 10 Gew.-%, bezogen auf die Gesamtmenge der schäumbaren Zusammensetzung, Bis-PEG/PPG-20/20 Dimethicone ist;
die Komponente c) 0,05 bis 2 Gew.-%, bezogen auf die Gesamtmenge der schäumbaren Zusammensetzung, PEG-Polymer ist, das aus der Gruppe bestehend aus PEG 7M bis PEG 45M ausgewählt ist; und
die optionale Komponente d) 0,05 bis 3 Gew.-%, bezogen auf die Gesamtmenge der schäumbaren Zusammensetzung, Ethylcellulose-Polymer ist.

6. Verwendung der alkoholischen Schaumzusammensetzung nach einem der Ansprüche 1 bis 5 als Desinfektionsmittel.

7. Verwendung der alkoholischen Schaumzusammensetzung nach einem der Ansprüche 1 bis 5 als Antiseptika zur Haut-, Stuhl- und Sputumdesinfektion sowie Instrumentendesinfektion.

8. Alkolholische Schaumzusammensetzung nach einem der Ansprüche 1 bis 5 zur verwendung als Antiseptika zur Wunddesinfektion.

9. Verwendung der alkoholischen Schaumzusammensetzung nach einem der Ansprüche 1 bis 5 als Wäsche- und Flächendesinfektionsmittel.

10. Verwendung der alkoholischen Schaumzusammensetzung nach einem der Ansprüche 1 bis 5 als Haut- und Händedesinfektionsmittel.

11. Desinfektionsmittel, umfassend: die alkoholische Schaumzusammensetzung nach einem der Ansprüche 1 bis 5.

12. Antiseptikum zur Wund-, Haut-, Stuhl- und Sputumdesinfektion oder Instrumentendesinfektion, umfassend: die alkoholische Schaumzusammensetzung nach einem der Ansprüche 1 bis 5.

13. Haut- und Händedesinfektionsmittel, umfassend: die alkoholische Schaumzusammensetzung nach einem der Ansprüche 1 bis 5.

## Claims

1. Alcoholic foam composition for disinfection comprising:
a) from 52 to ≤99% by weight, with respect to the total quantity of the foamable composition, of an alcohol which is selected from methanol, ethanol, 1-propanol, 2-propanol, 1-butanol, isobutyl alcohol, tert-butyl alcohol, the amyl alcohols 1-, 2-, 3-pentanol, neopentyl alcohol, 1-hexanol and admixtures thereof;
b) from 0.5 to 20% by weight, with respect to the total quantity of the foamable composition, of at least one silicone surfactant, wherein the silicone surfactant is selected from polysiloxane polyether copolymers;
c) from 0.01 to 3% by weight, with respect to the total quantity of the foamable composition, of at least one polyalkylene glycol;
d) at least one foam stabilising agent which is selected from the group comprising cellulose ether;
e) at least one element which is selected from the group comprising cosmetic additives, excipients, active ingredients and water or an admixture thereof, wherein the surface tension of the component b) is in the range ±15 dyn/cm of the surface tension of the component a) or corresponds to the surface tension of the component a), and
the total of the components a) to e) is 100% by weight with respect to the total quantity of the foam composition.

2. Alcoholic foam composition according to claim 1, wherein from 52 to ≤99% by weight, with respect to the total quantity of the composition, of ethanol is contained in the foam alone or as part of the component a).

3. Alcoholic foam composition according to claim 1 or 2, **characterized in that** at least 80 or 90% by weight, with respect to the total quantity of the foamable composition, of ethanol is contained in the foamable composition as component a).

4. Alcoholic foam composition according to any one of claims 1 to 3, **characterised in that** from 0.01 to 10% by weight, with respect to the total quantity of the foamable composition, of at least one foam stabilising agent is contained in the foamable composition as component d).

5. Alcoholic foam composition according to claim 1, wherein:
the component a) is from 55 to 96% by weight, with respect to the total quantity of the foamable composition, of ethanol;
the component b) is from 1 to 10% by weight, with respect to the total quantity of the foamable composition, of bis-PEG/PPG-20/20 dimethicone;
the component c) is from 0.05 to 2% by weight, with respect to the total quantity of the foamable composition, of PEG polymer, which is selected from the group comprising PEG 7M to PEG 45M; and
the optional component d) is from 0.05 to 3% by weight, with respect to the total quantity of the foamable composition, of ethyl cellulose polymer.

6. Use of the alcoholic foam composition according to any one of claims 1 to 5 as a disinfection agent.

7. Use of the alcoholic foam composition according to any one of claims 1 to 5 as antiseptics for skin, stool and sputum disinfection and instrument disinfection.

8. Alcoholic foam composition according to any one of claims 1 to 5 for use as antiseptics for wound disinfection.

9. Use of the alcoholic foam composition according to any one of claims 1 to 5 as a laundry and surface disinfection agent.

10. Use of the alcoholic foam composition according to any one of claims 1 to 5 as a skin and hand disinfection agent.

11. Disinfection agent comprising: the alcoholic foam composition according to any one of claims 1 to 5.

12. Antiseptic for wound, skin, stool and sputum disinfection or instrument disinfection, comprising: the alcoholic foam composition according to any one of claims 1 to 5.

13. Skin and hand disinfection agent comprising: the alcoholic foam composition according to any one of claims 1 to 5.

## Revendications

1. Composition de mousse contenant de l'alcool servant à désinfecter, comprenant :
a) 52 à ≤ 99 % en poids, par rapport à la quantité totale de la composition moussante, d'un alcool qui est choisi parmi le méthanol, l'éthanol, le 1-propanol, le 2-propanol, le 1-butanol, l'alcool isobutylique, l'alcool tert-butylique, les alcools amyliques, le 1-, 2-, 3-pentanol, l'alcool de néopentyle, le 1-hexanol et des mélanges de ceux-ci ;
b) 0,5 à 20 % en poids, par rapport à la quantité totale de la composition moussante, d'au moins un tensioactif à base de silicone, dans laquelle le tensioactif à base de silicone est choisi parmi des copolymères de polysiloxane-polyéther ;
c) 0,01 à 3 % en poids, par rapport à la quantité totale de la composition moussante, d'au moins un polyalkylène glycol ;
d) au moins un agent stabilisant de mousse qui est choisi parmi le groupe constitué d'éther cellulosique ;
e) au moins un élément qui est choisi parmi le groupe constitué d'additifs cosmétiques, d'excipients, de principes actifs et d'eau ou d'un mélange de ceux-ci, la tension surfacique du composant b) se situant dans la plage allant de ± 15 dyn/cm de la tension surfacique du composant a) ou correspond à la tension surfacique du composant a) et la somme des composants a) à e) est de 100 % en poids par rapport à la quantité totale de la composition de mousse.

2. Composition de mousse contenant de l'alcool selon la revendication 1, dans laquelle 52 à 99 % en poids, par rapport à la quantité totale de la composition, d'éthanol sont contenus seul ou en tant que partie du composant a) dans la mousse.

3. Composition de mousse contenant de l'alcool selon la revendication 1 ou 2, **caractérisée en ce qu'**au moins 80 ou 90 % en poids, par rapport à la quantité totale de la composition moussante, d'éthanol en tant que composant a) sont contenus dans la composition moussante.

4. Composition de mousse contenant de l'alcool selon l'une quelconque des revendications 1 à 3, **caractérisée en ce que** 0,01 à 10 % en poids, par rapport à la quantité totale de la composition moussante, d'au moins un agent stabilisant de mousse en tant que composant d) sont contenus dans la composition moussante.

5. Composition de mousse contenant de l'alcool selon la revendication 1, dans laquelle :
le composant a) est 55 à 96 % en poids, par rapport à la quantité totale de la composition moussante, d'éthanol ;
le composant b) est 1 à 10 % en poids, par rapport à la quantité totale de la composition moussante, de bis-PEG/PPG-20/20 diméthicone ;
le composant c) est 0,05 à 2 % en poids, par rapport à la quantité totale de la composition moussante, de polymère PEG qui est choisi parmi le groupe constitué du PEG 7M à PEG 45M ; et
le composant d) optionnel est 0,05 à 3 % en poids, par rapport à la quantité totale de la composition moussante, de polymère d'éthylcellulose.

6. Utilisation de la composition de mousse contenant de l'alcool selon l'une quelconque des revendications 1 à 5 en tant que produit désinfectant.

7. Utilisation de la composition de mousse contenant de l'alcool selon l'une quelconque des revendications 1 à 5 en tant qu'antiseptiques servant à désinfecter la peau, les fesses et la gorge ainsi que servant à désinfecter des instruments.

8. Composition de mousse contenant de l'alcool selon l'une quelconque des revendications 1 à 5 destinée à être utilisée en tant qu'antiseptique aux fins de la désinfection de plaies.

9. Utilisation de la composition de mousse contenant de l'alcool selon l'une quelconque des revendications 1 à 5 en tant que produit désinfectant pour le linge et de surfaces.

10. Utilisation de la composition de mousse contenant de l'alcool selon l'une quelconque des revendications 1 à 5 en tant que produit désinfectant pour la peau et les mains.

11. Produit désinfectant comprenant : la composition de mousse contenant de l'alcool selon l'une quelconque des revendications 1 à 5.

12. Antiseptique servant à désinfecter les plaies, la peau, les fesses et la gorge ou servant à désinfecter des instruments, comprenant : la composition de mousse contenant de l'alcool selon l'une quelconque des revendications 1 à 5.

13. Produit désinfectant pour la peau et les mains comprenant : la composition de mousse contenant de l'alcool selon l'une quelconque des revendications 1 à 5.
